(19) Europäisches Patentamt
European Patent Office
Office européen des brevets

(11) Publication number: 0 127 708
B1

(12) # EUROPEAN PATENT SPECIFICATION

(45) Date of publication of patent specification: 09.09.87

(51) Int. Cl.$^4$: C 07 D 215/20

(21) Application number: 83303202.2

(22) Date of filing: 03.06.83

(54) **Trans-dl-1-alkyl-6-alkoxyoctahydroquinolines.**

(43) Date of publication of application:
12.12.84 Bulletin 84/50

(45) Publication of the grant of the patent:
09.09.87 Bulletin 87/37

(84) Designated Contracting States:
BE CH DE FR IT LI LU NL SE

(56) References cited:
EP-A-0 013 788
EP-A-0 050 881

(73) Proprietor: ELI LILLY AND COMPANY
307, East McCarty Street
Indianapolis Indiana 46285 (US)

(72) Inventor: Schaus, John Mehnert
5427 North Delaware Street
Indianapolis Indiana 46220 (US)
Inventor: Garbrecht, William Lee
7303 Wood Stream Drive
Indianapolis Indiana (US)

(74) Representative: Crowther, Terence Roger et al
Erl Wood Manor
Windlesham Surrey GU20 6PH (GB)

EP 0 127 708 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

US—A—4,198,415 which corresponds to EP—A—50 881 discloses a group of octahydropyrazolo-[3,4-g]quinolines useful as prolactin inhibitors and in the treatment of Parkinsonism. The compounds disclosed therein are said to have dopamine agonist (dopaminergic) activity. One of the more active drugs disclosed carries an n-propyl group on the quinoline nitrogen.

A key intermediate in the preparation of these octahydropyrazolo[3,4-g]quinolines is a trans-dl-1-alkyl-6-oxadecahydroquinoline, one of whose stereoisomers (the 4aα, 8aβ compound) is disclosed in VII of column 5 of US—A—4,198,415. The same intermediate is used to prepare a related group of compounds, the 4,4a,5,6,7,8,8a,9-octahydro-2H-pyrrolo[3,4-g]quinolines of US—A—4,235,909 which corresponds to EP—A—13 788. The octahydro-2H-pyrrolo[3,4-g]quinolines are similar in activity to the octahydro-1H(and 2H)-pyrazolo[3,4-g]quinolines in that the compounds are dopamine agonists having activity as inhibitors of prolactin secretion and also in experimental models of Parkinson's disease.

The method of synthesizing the trans-dl-1-alkyl-6-oxodecahydroquinolines as disclosed in the two patents, while operative, involves five steps from a commercially available starting material. Furthermore, yields are not as high as desirable for a commercial process.

This invention concerns an improved method of preparing trans-dl-1-alkyl-6-oxodecahydroquinolines which is easier to carry out and which gives higher yields than the process previously known.

This invention concerns a novel key intermediate for preparing the trans-dl-1-alkyl-6-oxodecahydro-quinolines, namely a trans-octahydroquinoline compound of the formula

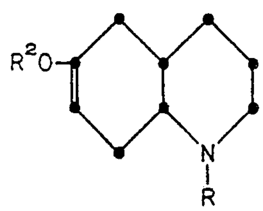

V

wherein R and $R^2$ are independently $C_1$—$C_3$ alkyl.

The trans racemic mixture of the compounds of formula V are shown by the structures below.

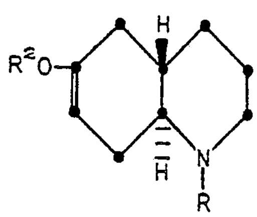

Va

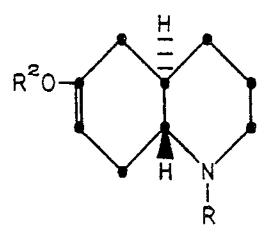

Vb

R and $R^2$ are defined as above.

The above octahydroquinoline compounds of formula V are prepared by reacting a hexahydroquinoline compound of the formula

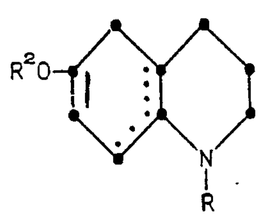

IV

where R and $R^2$ are defined as before and the dotted line represents the presence of one double bond, with a reducing agent of the formula $MBH_4$ or $MCNBH_3$ where M is an alkali metal, in an inert solvent.

Examples of the alkali metal (M) in the reducing agent are sodium, potassium, or lithium, preferably sodium. Preferred reducing agents are sodium borohydride or sodium cyanoborohydride. A critical characteristic of the reducing agent is its ability for stereoselective reduction. A mutual inert solvent is used such as tetrahydrofuran, dioxane, dimethoxyethane, diglyme or a $C_1$—$C_3$ alkanol, especially methanol or ethanol. Particularly preferred solvents are tetrahydrofuran, methanol, and ethanol. The preferred temperature is room temperature, about 20 to 25°C. Optionally, a small quantity of a mineral acid such as HCl or $H_2SO_4$, or of an $C_1$—$C_3$ alkanoic acid, preferably glacial acetic acid, may be present.

The isomeric mixture of the compounds of formula IV consists of the following compounds.

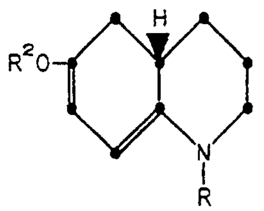

IVa

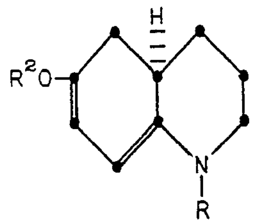

IVb

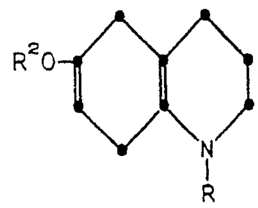

IVc

where R and $R^2$ are defined as before.

In the above structures, R and $R^2$ are $C_1$—$C_3$ alkyl such as methyl, ethyl and n-propyl.

The entire reaction sequence starting with a known compound, going through the claimed intermediate, and forming the pharmaceutically active products is shown in Flow Chart A in which the terms are defined as follows: R and $R^2$ are independently $C_1$—$C_3$ alkyl, X is a halogen or a pseudohalogen, M is an alkali metal, and $R^1$ is R or allyl.

## Flow Chart A

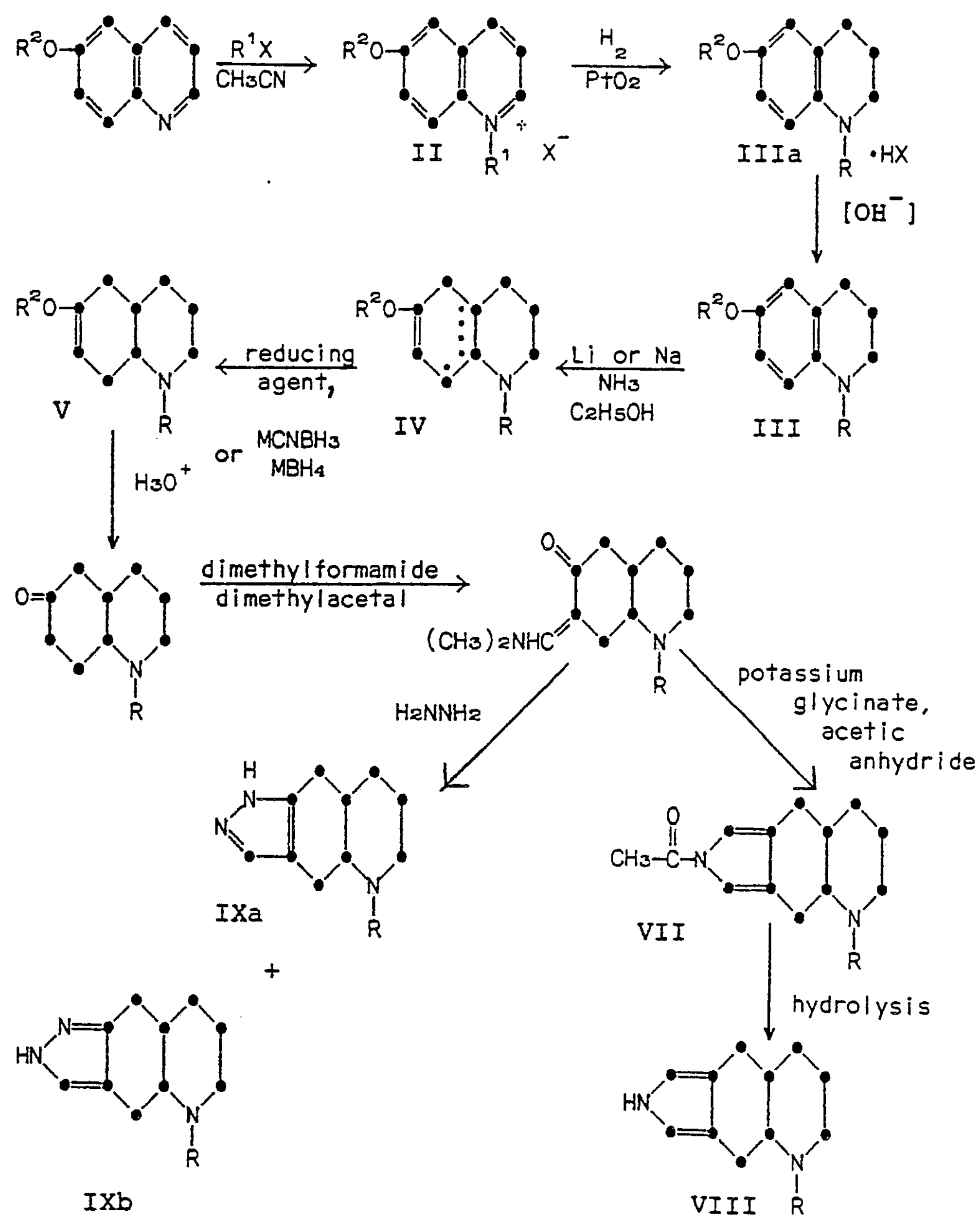


According to Flow Chart A, a 6-alkoxyquinoline such as 6-methoxyquinoline (available commercially), is quarternized with an alkyl or allyl halide or pseudo halide ($R^1X$), preferably an alkyl iodide (RI), in an inert solvent such as acetonitrile. The term "pseudo halide" here refers to such groups as mesyloxy or tosyloxy which behave chemically like a halide. The quaternization reaction is carried out conveniently at the boiling point of the solvent employed. The quarternary salt (II) is a crystalline material. In the second step of the synthetic procedure, this salt is hydrogenated under pressure using a noble metal catalyst such as platinum (supplied as $PtO_2$), palladium, rhodium and others. This hydrogenation is preferably carried out in an acidic medium such as glacial acetic acid and at elevated temperatures; i.e., in the range 60—100°C. Both low and high pressure reaction conditions are operative; i.e., pressures varying from 3.5 to 4.2 $kg/cm^2$ for low pressure hydrogenation to up to 70.3 $kg/cm^2$ for high pressure hydrogenation are operative. For example, about ten hours are required to reduce one-half mole of (II) to 1-alkyl-6-methoxy-1,2,3,4-tetrahydroquinoline, as the hydrogen iodide salt (IIIa) at 70.3 $kg/cm^2$ with a $PtO_2$ catalyst in glacial acetic acid.

The free base (III) is prepared from the salt by treatment of an aqueous solution of the salt with base followed by extraction of the base-insoluble tetrahydroquinoline into a water immiscible solvent.

In a second reduction step, the tetrahydroquinoline is reduced under Birch reduction conditions, using an alkali metal, such as sodium or lithium dissolved in liquid ammonia. The quinoline is customarily added

as a solution in THF to the solution of the alkali metal in liquid $NH_3$. After the reduction mixture is stirred at liquid ammonia temperatures for a suitable period of time, anhydrous ethanol is added until the blue color is discharged. The reaction mixture is then allowed to warm to room temperature as the $NH_3$ evaporates. The residual THF solution contains a mixture of hexahydroquinolines of formula IV [represented hereinbefore by IVa (1-alkyl-6-alkoxy-1,2,3,4,4aS,5-hexahydroquinoline), IVb (1-alkyl-6-alkoxy-1,2,3,4,4aR,5-hexahydroquinoline) and IVc (1-alkyl-6-alkoxy-1,2,3,4,5,8-hexahydroquinoline)]. This mixture of hexahydroquinoline isomers is then stereoselectively reduced using a reducing agent of the formula $MBH_4$ or $MCNBH_3$, where M is an alkali metal, such as sodium borohydride or sodium cyanoborohydride. A solvent such as THF or $C_1$—$C_3$ alkanol is employed in this reaction. Also, a small quantity of mineral acid such as HCl or $H_2SO_4$, or glacial acetic acid, or of an $C_1$—$C_3$ alkanoic acid may be added, preferably glacial acetic acid. The reduction is carried out at room temperature.

The product of the reduction is a racemate of formula V [represented hereinbefore by Va and Vb, where Va is named as a 1-alkyl-6-alkoxy-1,2,3,4,4aS,5,8,8aS-octahydroquinoline and Vb is named as a 1-alkyl-6-alkoxy-1,2,3,4,4aR,5,8,8aR-octahydroquinoline]. Treatment of this racemate with aqueous acid, preferably HCl, yields a racemic mixture of trans-dl-1-alkyl-6-oxadecahydroquinolines of formula I. The 6-oxo compound of I is reacted with dimethylformamide dimethylacetal to yield a trans-dl-1-alkyl-6-oxo-7-dimethylaminomethylene-decahydroquinoline (VI). The intermediate of formula (VI) can be further reacted in one of the following two ways. Reaction with potassium glycinate followed by acetic anhydride yields trans-dl-2-acetyl-5-alkyl-4,4a,5,6,7,8,8a,9-octahydro-2H-pyrrolo[3,4-g]quinoline (VII). The compound of formula VII then undergoes alkaline hydrolysis to yield trans-dl-5-alkyl-4,4a,5,6,7,8,8a,9-octahydropyrrolo-[3,4-g]quinolines (VIII), described in US—A—4,235,909. Secondly, the compound of formula (VI) reacts with hydrazine to yield a tautomeric mixture of trans-dl-5-alkyl-4,4a,5,6,7,8,8a,9-octahydro-1H(or 2H)-pyrazolo[3,4-g]quinoline (IXa and IXb), described in US—A—4,198,415.

Preparation of the above compounds is illustrated by the following specific Examples.

## Preparation of starting materials

### Preparation 1

Preparation of 1-n-propyl-6-methoxyquinolinium iodide.

Four hundred grams of 6-methoxyquinoline were dissolved in 2.5 l of acetonitrile containing 854.4 g of n-propyl iodide. The resulting solution was heated to reflux for about 18 hours under a nitrogen blanket. The reaction mixture was cooled and the solvent removed by evaporation *in vacuo*. The residue was dissolved in acetone and ether was added to the point of incipient precipitation. Crystals were produced by scratching. Crystalline 1-n-propyl-6-methoxyquinolinium iodide thus prepared was isolated by filtration; weight = 547.2 g; melting point = 111—115°C. $R_f$ (4:1 Chloroform:methanol, silica) = .58. An additional 136.2 g of desired product were obtained from the filtrate. NMR was compatible with the proposed structure.

### Preparation 2

Preparation of 1-n-Propyl-6-methoxy-1,2,3,4-tetrahydroquinoline.

1-n-Propyl-6-methoxyquinoline iodide, from Preparation 1, was hydrogenated to yield 1-n-propyl-6-methoxy-1,2,3,4-tetrahydroquinoline. In a typical run, 163 g of the quarternary salt were dissolved in 1917 ml of glacial acetic acid to which were added 20 g of platinum oxide. Hydrogenation was carried out at 70.3 kg/cm$^2$ at a temperature of about 100°C. After about ten hours, the theoretical amount of hydrogen had been absorbed. The hydrogenation mixture was then filtered to remove the catalyst and the solvent then removed from the filtrate by evaporation *in vacuo*. The resulting residue was dissolved in water and the aqueous solution made basic by the addition of saturated aqueous sodium bicarbonate. The aqueous layer was extracted with ether. The ether extracts containing 1-n-propyl-6-methoxy-1,2,3,4-tetrahydroquinoline formed in the above reaction were washed with water and then dried. The ether was removed by evaporation *in vacuo*. Ninety-two grams of 1-n-propyl-6-methoxy-1,2,3,4-tetrahydroquinoline were obtained; yield = 90.6%.

The above hydrogenation can also be carried out at a low pressure such as 4.22 kg/cm$^2$. In addition, in place of $PtO_2$, Pd-on-C or Rh on $Al_2O_3$ can also be used with equal or superior results.

Alternatively, an alkyl halide such as allyl bromide can be used to quarternize the 6-methoxyquinoline since hydrogenation of the N-allyl-6-methoxyquinolinium since hydrogenation of the N-allyl-6-methoxyquinolinium bromide with a noble metal catalyst such as $PtO_2$ will ultimately yield the identical 1-n-propyl-6-methoxy-1,2,3,4-tetrahydroquinoline.

### Preparation 3

Preparation of 1-n-Propyl-6-methoxy-1,2,3,4,5,8-hexahydroquinoline, 1-n-Propyl-6-methoxy-1,2,3,4,4aR,5-hexahydroquinoline and 1-n-Propyl-6-methoxy-1,2,3,4,4aS,5-hexahydroquinoline.

Three liters of ammonium were dried over sodium metal for about one hour. 800 ml of ammonia thus dried were then distilled into a 3 l three-neck flask equipped with gas inlet tube, condenser with drying tube attached and additional funnel. The ammonia was stirred with a magnetic stirrer. A solution containing 40 g of 1-n-propyl-6-methoxy-1,2,3,4-tetrahydroquinoline, prepared as Preparation 2, in 200 ml of THF (dry, distilled) were added. Ten g of lithium metal were cut into chunks of about 1 cm$^3$ size and these chunks

were added in a single batch to the liquid ammonia-tetrahydroquinoline-THF solution. The reaction mixture was stirred for about 20 minutes. Anhydrous ethanol (about 160 ml) was added in dropwise fashion over a 15 minute period. The resulting mixture was allowed to stir overnight under a nitrogen atmosphere without external cooling. During this time, the ammonia evaporated. Four-hundred ml of water was then added. The aqueous mixture was extracted with three 200 ml portions of dichloromethane. The dichloromethane layers were combined and the combined layers washed with 250 ml of saturated aqueous sodium chloride. The dichloromethane layers were then dried and the solvent removed by evaporation *in vacuo*. The mixture of 1-n-propyl-6-methoxy-1,2,3,4,5,8-hexahydroquinoline, 1-n-propyl-6-methoxy-1,2,3,4,4aR,5-hexahydroquinoline and 1-n-propyl-6-methoxy-1,2,3,4,4aS,5-hexahydroquinoline thus produced distilled in the range 84—120°C at a pressure of 4Pa (= 0.03 Torr); yield = 32.6 g. (80.5%).

Preparation of Final Products

Example 1

Preparation of trans-dl-1-n-Propyl-6-methoxy-1,2,3,4,4a,5,8,8a-octahydroquinoline.

A solution was prepared containing 4.4 g of sodium cyanoborohydride in 250 ml of dried, distilled THF. 14.8 g of a mixture of 1-n-propyl-6-methoxy-1,2,3,4,5,8-hexahydroquinoline, 1-n-propyl-6-methoxy-1,2,3,4,4aR,5-hexahydroquinoline and 1-n-propyl-6-methoxy-1,2,3,4,4aS,5-hexadroquinoline, prepared in Preparation 3, in 100 ml of THF were then added, followed by 1.7 ml of glacial acetic acid. The reaction mixture was stirred at ambient temperature for about 1.25 hours, at which time another 1.7 ml of glacial acetic acid were added. Stirring was continuous for an additional 30 minutes. The entire reaction mixture was then poured into about 300 ml of water. The aqueous mixture was extracted three times with 200 ml of portions of dichloromethane. The organic layers were combined, the combined layers were washed once with an equal volume of water and were then dried. The solvent was removed by evaporation *in vacuo* and the residual yellow viscous oil was distilled. Trans-dl-1-n-propyl-6-methoxy-1,2,3,4,4a,5,8,8a-octahydroquinoline thus prepared distilled in the range 70—140°C at 20-26.6 Pa (= 0.15—0.20 Torr) giving a total weight of 10 g. (66% yield).

Example 2

Preparation of trans-dl-1-n-Propyl-6-methoxy-1,2,3,4,4a,5,8,8a-octahydroquinoline.

When the procedure of Example 1 was repeated using 10 g of a mixture of 1-n-propyl-6-methoxy-1,2,3,4,5,8-hexahydroquinoline, 1-n-propyl-6-methoxy-1,2,3,4,4aR,5-hexahydroquinoline, and 1-n-propyl-6-methoxy-1,2,3,4,4aS,5-hexahydroquinoline in 140 ml of ethanol, 2.19 g of sodium cyanoborohydride and 2 ml of HCl, there was obtained trans-dl-1-n-propyl-6-methoxy-1,2,3,4,4a,5,8,8a-octahydroquinoline (95% yield) having the same boiling point range as Example 1.

Example 3

Preparation of trans-dl-1-n-Propyl-6-methoxy-1,2,3,4,4a,5,8,8a-octahydroquinoline.

To a solution of 10 g of a mixture of 1-n-propyl-6-methoxy-1,2,3,4,5,8-hexahydroquinoline, 1-n-propyl-6-methoxy-1,2,3,4,4aR,5-hexahydroquinoline and 1-n-propyl-6-methoxy-1,2,3,4,4aS,5-hexahydroquinoline in 140 ml of ethanol was added, at one time, 2.5 ml of glacial acetic acid. A solution of 1.32 g of sodium borohydride in ethanol was prepared and added portionwise to the first solution. The reaction mixture was cooled and stirred under nitrogen about 16 hours. The product, trans-dl-1-n-propyl-6-methoxy-1,2,3,4,4a,5,8,8a-octahydroquinoline, was isolated according to the procedure of Example 1 and was identical to the product of Example 1. (82% yield).

Example 4

Preparation of trans-dl-1-n-Propyl-6-methoxy-1,2,3,4,4a,5,8,8a-octahydroquinoline.

When the procedure of Example 3 was repeated using 140 ml of isopropanol for the ethanol, there was obtained trans-dl-1-n-propyl-6-methoxy-1,2,3,4,4a,5,8,8a-octahydroquinoline (52% yield) identical to the product of Example 1.

Example 5

Preparation of trans-dl-1-n-Propyl-6-methoxy-1,2,3,4,4a,5,8,8a-octahydroquinoline.

When the procedure of Example 3 was repeated using 140 ml of methanol for the ethanol, there was obtained trans-dl-1-n-propyl-6-methoxy-1,2,3,4,4a,5,8,8a-octahydroquinoline (92% yield) identical to the product of Example 1.

Example 6

Preparation of trans-dl-1-n-propyl-6-methoxy-1,2,3,4,4a,5,8,8a-octahydroquinoline.

When the procedure of Example 3 was repeated using 40 ml of ethanol to dissolve the sodium borohydride and 100 ml of methanol for the 140 ml of ethanol, there was obtained trans-dl-1-n-propyl-6-methoxy-1,2,3,4,4a,5,8,8a-octahydroquinoline (98% yield) identical with the product of Example 1.

Example 7

Preparation of trans-dl-1-n-Propyl-6-methoxy-1,2,3,4,4a,5,8,8a-octahydroquinoline.

When the procedure of Example 6 was repeated omitting the glacial acetic acid, there was obtained trans-dl-1-n-propyl-6-methoxy-1,2,3,4,4a,5,8,8a-octahydroquinoline (89% yield) identical with the product of Example 1.

Example 8

Preparation of trans-dl-1-n-Propyl-6-methoxy-1,2,3,4,4a,5,8,8a-octahydroquinoline.

When the procedure of Example 3 was repeated using 10 g of the mixture of 1-n-propyl-6-methoxy-hexahydroquinolines, 1.07 g of sodium borohydride in 40 ml of ethanol, 100 ml of methanol, and 2.8 ml of glacial acetic acid, there was obtained trans-dl-1-n-propyl-6-methoxy-1,2,3,4,4a,5,8,8a-octahydroquinoline (99% yield) identical to the product of Example 1.

Preparation of Active Products

Example A

Preparation of trans-dl-1-n-propyl-6-oxo-1,2,3,4,4a,5,6,7,8,8a-decahydroquinoline.

A solution was prepared from 3.1 g of trans-dl-1-n-propyl-6-methoxy-1,2,3,4,4a,5,8,8a-octahydroquinoline in 25 ml of THF, from Example 1. Four ml of 10% aqueous sulfuric acid were added. The resulting two-phase mixture was heated at refluxing temperature for about 17 hours after which time it was poured into dilute aqueous sodium hydroxide. The alkaline aqueous mixture was extracted with dichloromethane. The dichloromethane extract was dried and the solvent removed *in vacuo*. A residual oil weighing about 2.8 g comprising trans-dl-1-n-propyl-6-oxodecahydroquinoline distilled in the range 63—87°C at 0.1 Torr. TLC indicated that the combined fractions contained in excess of 90% trans-dl-1-n-propyl-6-oxodecahydroquinoline.

Alternatively, 5.0 g of trans-dl-1-n-propyl-6-methoxy-1,2,3,4,4a,5,8,8a-octahydroquinoline were dissolved in 50 ml of THF. Twenty-five ml of 1N aqueous hydrochloric acid were added and the mixture stirred for one-half hour under a nitrogen atmosphere. The reaction mixture was then made basic with 14N aqueous ammonium hydroxide and the alkaline mixture extracted three times with dichloromethane. The organic extracts were combined, dried and the solvent removed *in vacuo*. 4.8 g of an orange transparent oil remained as a residue. TLC indicated a single spot at $R_f$ = .67 with slight leading and trailing spots as impurities. Distillation yielded trans-dl-1-n-propyl-6-oxodecahydroquinoline boiling at 77—87°C at 0.025 Torr. total yield = 4.39 g (94.1%).

As previously stated, the trans-dl-1-alkyl-6-oxodecahydroquinolines (I), can be reacted with dimethylformamide dimethylacetal to yield a trans-dl-1-alkyl-6-oxo-7-dimethylaminomethylene decahydroquinoline (VI). Reaction of this intermediate with potassium glycinate followed by treatment with acetic anhydride yields a trans-dl-2-acetyl-5-alkyl-4,4a,5,6,7,8,8a,9-octahydro-2H-pyrrolo[3,4-g]quinoline (VII). Alkaline hydrolysis of the acetyl derivative yields trans-dl-5-alkyl-4,4a,5,6,7,8,8a,9-octahydropyrrolo[3,4-g]quinoline (VIII), a dopamine agonist useful in treating Parkinsonism or excessive prolactin secretion (See US—A—4,235,909). Alternatively, the 7-dimethylaminomethylene commpound (VI) reacts with hydrazine to yield a tautomeric mixture consisting of trans-dl-5-alkyl-4,4a,5,6,7,8,8a,9-octahydro-1H-pyrazolo[3,4-g]quinoline and the corresponding 2H compound (IXa and IXb), useful also as dopamine agonists. (See US—A—4,198,415).

**Claims**

1. A trans-octahydroquinoline compound of the formula

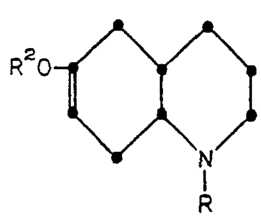

V

where R and $R^2$ are independently $C_1$—$C_3$ alkyl.

2. Trans-dl-1-n-propyl-6-methoxy-1,2,3,4,4a,5,8,8a-octahydroquinoline.

3. A process for preparing a trans-octahydroquinoline compound of the formula

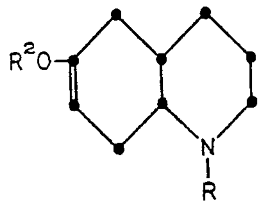

V

as defined in claim 1, wherein R and $R^2$ are independently $C_1$—$C_3$ alkyl, which comprises reacting a hexahydroquinoline compound of the formula

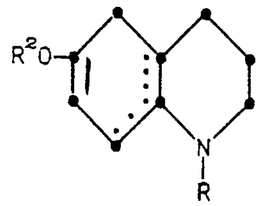

IV

wherein R and $R^2$ are defined as before and the dotted line represents the presence of one double bond with a reducing agent of the formula $MCNBH_3$ or $MBH_4$ where M is an alkali metal, in an inert solvent.

4. The process of claim 3 wherein the reducing agent is sodium cyanoborohydride.

5. The process of claim 4 wherein the inert solvent is tetrahydrofuran.

6. The process of claim 3 wherein the reducing agent is sodium borohydride.

7. The process of claim 6 wherein the inert solvent is methanol or ethanol.

8. The process of claim 3, 4 or 6 wherein the mineral acid is present.

9. The process of claim 3, 4 or 6 wherein glacial acetic acid is present.

10. The process of claim 3, 4 or 9 for preparing trans-dl-1-n-propyl-6-methoxy-1,2,3,4,4a,5,8,8a-octahydroquinoline which comprises reacting a mixture of 1-n-propyl-6-methoxy-1,2,3,4,4a,5,8,-hexahydroquinoline, 1-n-propyl-6-methoxy-1,2,3,4,4aR,5-hexahydroquinoline and 1-n-propyl-6-methoxy-1,2,3,4,4aS,5-hexahydroquinoline with sodium cycanoborohydride and glacial acetic acid.

11. The process of claim 3, 6 or 9 for preparing trans-dl-1-n-propyl-6-methoxy-1,2,3,4,4a,5,8,8a-octahydroquinoline which comprises reacting a mixture of 1-n-propyl-6-methoxy-1,2,3,4,5,8-hexahydroquinoline, 1-n-propyl-6-methoxy-1,2,3,4,4aR,5-hexahydroquinoline and 1-n-propyl-6-methoxy-1,2,3,4,4aS,5-hexahydroquinoline with sodium borohydride, and glacial acetic acid.

**Patentansprüche**

1. trans-Octahydrochinolinverbindung der Formel

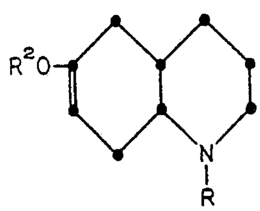

V

worin R und $R^2$ unabhängig für $C_1$ bis $C_3$-Alkyl stehen.

2. trans-dl-1-n-Propyl-6-methoxy-1,2,3,4,4a,5,8,8a-octahydrochinolin.

3. Verfahren zur Herstellung einer trans-Octahydrochinolinverbindung der Formel

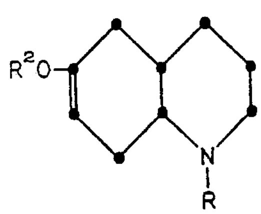

V

gemäß Anspruch 1, worin R und $R^2$ unabhängig für $C_1$ bis $C_3$-Alkyl stehen, dadurch gekennzeichnet, daß man eine Hexahydrochinolinverbindung der Formel

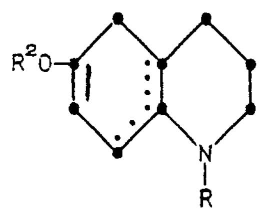

IV

worin R und $R^2$ wie oben definiert sind und die punktierte Linie die Anwesenheit einer Doppelbindung zeigt, mit einem Reduktionsmittel der Formel $MCNBH_3$ oder $MBH_4$, worin M ein Alkalimetall ist, in einem inerten Lösungsmittel umsetzt.

4. Verfahren nach Anspruch 3, dadurch gekennzeichnet, daß das Reduktionsmittel Natriumcyanoborhydrid ist.

5. Verfahren nach Anspruch 4, dadurch gekennzeichnet, daß das inerte Lösungsmittel Tetrahydrofuran ist.

6. Verfahren nach Anspruch 3, dadurch gekennzeichnet, daß das Reduktionsmittel Natriumborhydrid ist.

7. Verfahren nach Anspruch 6, dadurch gekennzeichnet, daß das inerte Lösungsmittel Methanol oder Ethanol ist.

8. Verfahren nach Anspruch 3, 4 oder 6, dadurch gekennzeichnet, daß eine Mineralsäure vorhanden ist.

9. Verfahren nach Anspruch 3, 4 oder 6, dadurch gekennzeichnet, daß Eisessigsäure vorhanden ist.

10. Verfahren nach Anspruch 3, 4 oder 9 zur Herstellung von trans-dl-1-n-Propyl-6-methoxy-1,2,3,4,4a,5,8,8a-octahydrochinolin, dadurch gekennzeichnet, daß man ein Gemisch von 1-n-Propyl-6-methoxy-1,2,3,4,5,8-hexahydrochinolin, 1-n-Propyl-6-methoxy-1,2,3,4,4aR,5-hexahydrochinolin und 1-n-Propyl-6-methoxy-1,2,3,4,4aS,5-hexahydrochinolin mit Natriumcyanoborhydrid und Eisessigsäure umsetzt.

11. Verfahren nach Anspruch 3, 6 oder 9 zur Herstellung von trans-dl-1-n-Propyl-6-methoxy-1,2,3,4,4a,5,8,8a-octahydrochinolin, dadurch gekennzeichnet, daß man ein Gemisch von 1-n-Propyl-6-methoxy-1,2,3,4,5,8-hexahydrochinolin, 1-n-Propyl-6-methoxy-1,2,3,4,4aR,5-hexahydrochinolin und 1-n-Propyl-6-methoxy-1,2,3,4,4aS,5-hexahydrochinolin mit Natriumborhydrid und Eisessigsäure umsetzt.

**Revendications**

1. Composé de trans-octahydroquinoléine de formule:

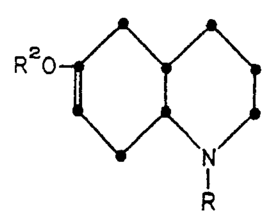

V

dans laquelle R et $R^2$ représentent chacun indépendamment l'un de l'autre un groupe alkyle en $C_1$—$C_3$.

2. La trans-dl-1-n-propyl-6-méthoxy-1,2,3,4,4a,5,8,8a-octahydroquinoléine.

3. Procédé de préparation d'un composé de trans-octahydroquinoléine de formule:

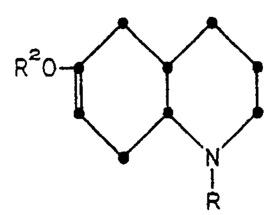

V

comme défini dans la revendication 1 où R et $R^2$ représentent chacun indépendamment l'un de l'autre un groupe alkyle en $C_1$—$C_3$, caractérisé en ce qu'il consiste à faire réagir un composé d'hexahydroquinoléine de formule:

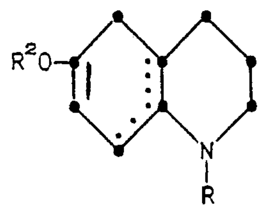

IV

dans laquelle R et $R^2$ ont les significations définies ci-dessus, tandis que la ligne en pointillés représente la présence d'une double liaison, avec un agent réducteur de formule $MCNBH_3$ ou $MBH_4$ où M représente un métal alcalin, dans un solvant inerte.

4. Procédé selon la revendication 3, caractérisé en ce que l'agent réducteur est le cyanoborohydrure de sodium.

5. Procédé selon la revendication 4, caractérisé en ce que le solvant inerte est le tétrahydrofuranne.

6. Procédé selon la revendication 3, caractérisé en ce que l'agent réducteur est le borohydrure de sodium.

7. Procédé selon la revendication 6, caractérisé en ce que le solvant inerte est le méthanol ou l'éthanol.

8. Procédé selon la revendication 3, 4 ou 6, caractérisé en ce qu'un acide minéral est présent.

9. Procédé selon la revendication 3, 4 ou 6, caractérisé en ce que l'acide acétique glacial est présent.

10. Procédé selon la revendication 3, 4 ou 9, pour la préparation de la trans-dl-1-n-propyl-6-méthoxy-1,2,3,4,4a,5,8,8a-octahydroquinoléine, caractérisé en ce qu'il consiste à faire réagir un mélange de la 1-n-propyl-6-méthoxy-1,2,3,4,5,8-hexahydroquinoléine, de la 1-n-propyl-6-méthoxy-1,2,3,4,4aR,5-hexahydroquinoléine et de la 1-n-propyl-6-méthoxy-1,2,3,4,4aS,5-hexahydroquinoléine avec le cyanoborohydrure de sodium et l'acide acétique glacial.

11. Procédé selon la revendication 3, 6 ou 9 pour la préparation de la trans-dl-1-n-propyl-6-méthoxy-1,2,3,4,4a,5,8,8a-octahydroquinoléine, caractérisé en ce qu'il consiste à faire réagir un mélange de la 1-n-propyl-6-méthoxy-1,2,3,4,5,8-hexahydroquinoléine, de la 1-n-propyl-6-méthoxy-1,2,3,4,4aR,R-hexahydroquinoléine et de la 1-n-propyl-6-méthoxy-1,2,3,4,4aS,5-hexahydroquinoléine avec le borohydrure de sodium et l'acide acétique glacial.